# EUROPÄISCHE PATENTANMELDUNG

(11) **EP 0 950 666 A1**
(43) Veröffentlichungstag der Anmeldung: **20.10.1999**
(21) Anmeldenummer: 99106249.8
(22) Anmeldetag: 14.04.1999
(51) Int. Cl.: C07K 16/08, C12N 5/20, G01N 33/569, G01N 33/577, A61K 39/42, A61K 39/245, C07K 14/03

(54) **Diagnostik und Therapie von Erkrankungen in Zusammenhang mit HHV-8-Infektionen durch anti-Kaposin Antikörpern oder durch Kaposin-Peptide**

(30) Priorität: 15.04.1998 DE 19816732
(71) Anmelder: GSF-Forschungszentrum für Umwelt und Gesundheit, GmbH, 85764 Oberschleissheim (DE)
(72) Erfinder: Haas, Jürgen, Dr., 80689 München (DE); Kremmer, Elisabeth, Dr., 85354 Freising (DE); Kliche, Stefanie, Dr., 81369 München (DE)
(74) Vertreter: Reinhard - Skuhra - Weise & Partner

(57) **Zusammenfassung**

Die vorliegende Erfindung betrifft monoklonale Antikörper, die spezifisch an eine antigene Determinante (Epitop) des Kaposin-Proteins oder eines Derivats hiervon binden können und sie erkennen, Hybridom-Zellinien, die diese monoklonalen Antikörper produzieren, Diagnostik-Systeme zum Nachweis auf das Vorliegen eines Kaposin-Proteins oder eines Derivats hiervon sowie von Antikörpern, die gegen das Kaposin-Protein oder ein Derivat hiervon gerichtet sind, Verfahren zum Nachweis der Expression des Kaposin-Proteins oder eines Derivats hiervon in einer biologischen Probe, Verfahren zum nachweis von Antikörpern, die gegen das Kaposin-Protein oder ein Derivat hiervon gerichtet sind, Verwendungen der erfindungsgemäß bereitgestellten monoktonalen Antikörper und Verwendungen des Kaposin-Proteins oder eines Derivats hiervon, je zur Diagnostik und Therapie.

SEQUENZPROTOKOLL

<110> GSF - Gesellschaft für Umwelt und Gesundheit GmbH

<120> Diagnostik und Therapie im Zusammenhang mit
      HHV-8-Infektionen.

<130> P10214

<140> DE 198 16 732.6
<141> 1999-04-15

<150> DE 198 16 732.6
<151> 1998-04-15

<160> 24

<170> PatentIn Ver. 2.1

<210> 1
<211> 60
<212> PRT
<213> Human Herpes Virus 8 (HHV-8)

<400> 1
Met Asp Arg Gly Leu Thr Val Phe Val Ala Val His Val Pro Asp Val
  1               5                  10                  15

Leu Leu Asn Gly Trp Arg Trp Arg Leu Gly Ala Ile Pro Pro Leu Val
             20                  25                  30

Cys Leu Leu Ala Ile Ser Val Val Pro Pro Ser Gly Gln Arg Gly Pro
         35                  40                  45

Val Ala Phe Arg Thr Arg Val Ala Thr Gly Ala His
     50                  55                  60

<210> 2
<211> 26
<212> PRT
<213> Human Herpes Virus 8 (HHV-8)

<400> 2
Met Asp Arg Gly Leu Thr Val Phe Val Ala Val His Val Pro Asp Val
  1               5                  10                  15

Leu Leu Asn Gly Trp Arg Trp Arg Leu Gly

            20                  25

<210> 3
<211> 16
<212> PRT
<213> Human Herpes Virus 8 (HHV-8)

<400> 3
Ala Ile Pro Pro Leu Val Cys Leu Leu Ala Ile Ser Val Val Pro Pro
1               5                   10                  15

<210> 4
<211> 18
<212> PRT
<213> Human Herpes Virus 8 (HHV-8)

<400> 4
Ser Gly Gln Arg Gly Pro Val Ala Phe Arg Thr Arg Val Ala Thr Gly
1               5                   10                  15

Ala His

<210> 5
<211> 10
<212> PRT
<213> Human Herpes Virus 8 (HHV-8)

<400> 5
Met Asp Arg Gly Leu Thr Val Phe Val Ala
1               5                   10

<210> 6
<211> 10
<212> PRT
<213> Human Herpes Virus 8 (HHV-8)

<400> 6
Leu Thr Val Phe Val Ala Val His Val Pro
1               5                   10

<210> 7
<211> 10

<212> PRT
<213> Human Herpes Virus 8 (HHV-8)

<400> 7
Val Ala Val His Val Pro Asp Val Leu Leu
1               5                   10

<210> 8
<211> 10
<212> PRT
<213> Human Herpes Virus 8 (HHV-8)

<400> 8
His Val Pro Asp Val Leu Leu Asn Gly Trp
1               5                   10

<210> 9
<211> 10
<212> PRT
<213> Human Herpes Virus 8 (HHV-8)

<400> 9
Asp Val Leu Leu Asn Gly Trp Arg Trp Arg
1               5                   10

<210> 10
<211> 10
<212> PRT
<213> Human Herpes Virus 8 (HHV-8)

<400> 10
Leu Asn Gly Trp Arg Trp Arg Leu Gly Ala
1               5                   10

<210> 11
<211> 10
<212> PRT
<213> Human Herpes Virus 8 (HHV-8)

<400> 11
Trp Arg Trp Arg Leu Gly Ala Ile Pro Pro
1               5                   10

<210> 12
<211> 10
<212> PRT
<213> Human Herpes Virus 8 (HHV-8)

<400> 12
Arg Leu Gly Ala Ile Pro Pro Leu Val Cys
1               5                   10

<210> 13
<211> 10
<212> PRT
<213> Human Herpes Virus 8 (HHV-8)

<400> 13
Ala Ile Pro Pro Leu Val Cys Leu Leu Ala
1               5                   10

<210> 14
<211> 10
<212> PRT
<213> Human Herpes Virus 8 (HHV-8)

<400> 14
Pro Leu Val Cys Leu Leu Ala Ile Ser Val
1               5                   10

<210> 15
<211> 10
<212> PRT
<213> Human Herpes Virus 8 (HHV-8)

<400> 15
Cys Leu Leu Ala Ile Ser Val Val Pro Pro
1               5                   10

<210> 16
<211> 10
<212> PRT
<213> Human Herpes Virus 8 (HHV-8)

<400> 16
Ala Ile Ser Val Val Pro Pro Ser Gly Gln
1               5                   10

<210> 17
<211> 10
<212> PRT
<213> Human Herpes Virus 8 (HHV-8)

<400> 17
Val Val Pro Pro Ser Gly Gln Arg Gly Pro
1               5                   10

<210> 18
<211> 10
<212> PRT
<213> Human Herpes Virus 8 (HHV-8)

<400> 18
Pro Ser Gly Gln Arg Gly Pro Val Ala Phe
1               5                   10

<210> 19
<211> 10
<212> PRT
<213> Human Herpes Virus 8 (HHV-8)

<400> 19
Gln Arg Gly Pro Val Ala Phe Arg Thr Arg
1               5                   10

<210> 20
<211> 10
<212> PRT
<213> Human Herpes Virus 8 (HHV-8)

<400> 20
Gly Pro Val Ala Phe Arg Thr Arg Val Ala
1               5                   10

<210> 21
<211> 10
<212> PRT
<213> Human Herpes Virus 8 (HHV-8)

<400> 21
Val Ala Phe Arg Thr Arg Val Ala Thr Gly
1               5                   10

<210> 22
<211> 10
<212> PRT
<213> Human Herpes Virus 8 (HHV-8)

<400> 22
Phe Arg Thr Arg Val Ala Thr Gly Ala His
1               5                   10

<210> 23
<211> 14
<212> PRT
<213> Human Herpes Virus 8 (HHV-8)

<400> 23
Gln Arg Gly Pro Val Ala Phe Arg Thr Arg Val Ala Thr Gly
1               5                   10

<210> 24
<211> 12
<212> PRT
<213> Human Herpes Virus 8 (HHV-8)

<400> 24
Gln Arg Gly Pro Val Ala Phe Arg Thr Arg Val Ala
1               5                   10

## Beschreibung

Die vorliegende Erfindung betrifft monoklonale Antikörper, die spezifisch eine antigene Determinante (Epitop) des Kaposin-Proteins oder eines Derivats hiervon erkennen und an sie binden können, Hybridom-Zellinien, die diese monoklonalen Antikörper produzieren, Diagnostik-Systeme zum Nachweis auf das Vorliegen eines Kaposin-Proteins oder eines Derivats hiervon sowie von monoklonalen Antikörpern, die gegen das Kaposin-Protein oder ein Derivat hiervon gerichtet sind, Verfahren zum Nachweis der Expression des Kaposin-Proteins oder eines Derivats hiervon in einer biologischen Probe, Verfahren zum Nachweis von Antikörpern, die gegen das Kaposin-Protein oder ein Derivat hiervon gerichtet sind, Verwendungen der erfindungsgemäß bereitgestellten monoklonalen Antikörper und Verwendungen des Kaposin-Proteins oder eines Derivats hiervon, je zur Diagnostik und Therapie.

Das Humane Herpesvirus 8 wurde in allen Formen des Kaposi Sarkoms, in Primären Effusionslymphomen (PEL), in der Castleman'schen Erkrankung, in Angiosarkomen, in Hautläsionen bei Transplantierten, in Plasmozytomen, bei Sarkoidose sowie in gesunden Kontrollpersonen nachgewiesen (Chang et al., 1994; Boshoff and Weiss, 1997). Seroepidemiologische Studien zeigten, daß HHV-8 in Nord- und Mitteleuropa sowie in Nordamerika im wesentlichen auf Risikogruppen beschränkt ist, und daß es große geographische und Altersunterschiede gibt. Diese Studien konnten darüber hinaus zeigen, daß bei Patienten mit Kaposi Sarkom Monate bis Jahre vor der Diagnose des Kaposi Sarkoms eine Serokonversion für HHV-8 feststellbar ist, und daß HHV-8 wahrscheinlich überwiegend durch Geschlechtsverkehr übertragen wird. Die klinischen Zeichen einer primären HHV-8 Infektion sind bisher unbekannt. Aufgrund des Nachweises von HHV-8 in praktisch 100% aller Kaposi Sarkome, der Korrelation zwischen der regionalen Seroprävalenz und der Inzidenz von HHV-8, und der Serokonversion vor dem Auftreten des Kaposi Sarkoms, wird heute davon ausgegangen, daß HHV-8 zumindest ein Kofaktor in der Tumorgenese des KS darstellt. Die Rolle von HHV-8 bei den anderen oben genannten Erkrankungen ist bisher ebenso unklar wie die Frage, ob HHV-8 bei weiteren, bisher nicht identifizierten Erkrankungen involviert ist.

HHV-8 gehört taxonomisch aufgrund Sequenzhomologie zur Unterfamilie der Gammaherpesviren und ist eng verwandt mit EBV und *Herpesvirus saimiri*. Das Genom von HHV-8 besitzt eine Größe von etwa 140 kB und wird flankiert von mehreren repetitiven Sequenzen mit einer Länge von ungefähr 800 bp (Russo et al., 1996). HHV-8 kodiert für etwa 80 Proteine, von denen etwa 10 eine Homologie zu zellulären Genprodukten besitzen (Neipel et al., 1997). Wie alle Herpesviren ist HHV-8 in der Lage, eine lytische Infektion zu verursachen, die anschließend in eine latente Infektion übergeht. In der Latenzphase werden wenigstens zwei virale Transkripte exprimiert, eine differentiell gsplicte mRNA, die für die drei viralen Proteihne v-cyclin, v-flip und LANA kodiert, sowie T0.7, eine kurze RNA mit 0,7 kb Länge und bisher unbekannter Funktion (Zhong et al., 1996). Das virale Transkript T0.7 ist die häufigste in der Latenzphase exprimierte RNA und besitzt zwei offene Leserahmen, die 60, 35 bzw. 47 Aminosäuren entsprechen.

Die HHV-8 Infektion wurde bisher hauptsächlich mittels Polymerase-Ketten-Reaktion unter Verwendung von HHV-8 spezifischen Oligonukleotid Primern nachgewiesen. Dieses direkte Nachweisverfahren hat die Nachteile, daß es (i) aufwendig und teuer ist (Preis ca. das fünf-bis zehnfache eines serologischen Nachweises), (ii) anfällig ist für falsch positive Ergebnisse durch Kontamination, (iii) nur akute, nicht aber früher durchgemachte Infektionen erfaßt und (iv) bei Durchführung aus dem peripheren Blut nur etwa 50% aller (akut) Infizierten erfaßt.

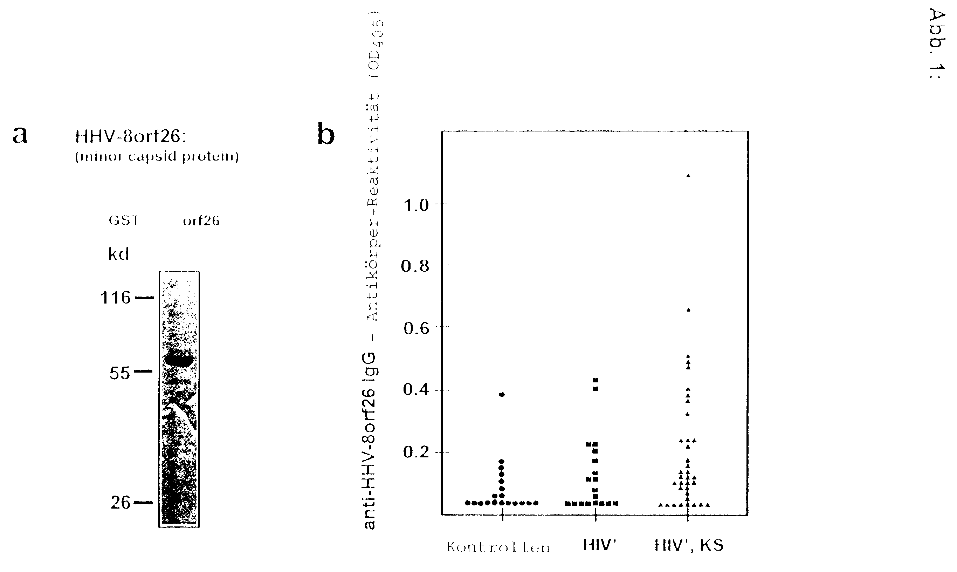

Die bisher entwickelten serologischen Nachweismethoden basieren entweder auf der Verwendung HHV-8 infizierter Zellinien oder rekombinanter viraler Proteine. Tests, die gegen HHV-8 gerichtete Antikörper auf HHV-8 positiven Zellinien mittels Immunfluoreszenz nachweisen, besitzen die Nachteile, daß sie wenig reproduzierbar sind (unter anderem weil die Kulturbedingungen für die HHV-8+ Zellinien nicht absolut konstant gehalten werden können), (ii) nicht maschinell auswertbar sind und sich daher für eine größere Zahl von Tests schlecht eignen und (iii) eine Kreuzreaktivität von Antikörpern gegen andere Viren teilweise schlecht auszuschließen ist. Ein gemeinsames Problem aller Tests, die auf rekombinanten viralen Proteinen basieren, ist die geringe Sensitivität. Hintergrund dieses Problems ist, daß nur gegen bestimmte Proteine des Virus Antikörper und in verschiedenen Individuen zum Teil Antikörper gegen unterschiedliche Proteine gemacht werden. Die bisher auf ihre Eignung in der serologischen Diagnostik getesteten Virusproteine besitzen eine Sensitivität zwischen ca. 30 und 80%. In Abbildung 1 wird als Beispiel gezeigt, daß nur in etwa 30% der KS Patienten Antikörper gegen das virale *minor capsid protein* VP23 nachweisbar sind (Abb. 1). Mit großer Wahrscheinlichkeit ist zur Entwicklung sensitiverer Tests die Verwendung mehrerer viraler Proteine notwendig.

Da das Kaposi Sarkom der dritthäufigste Tumor nach Organtransplantationen ist und nach dem heutigen Kenntnisstand eine enge Assoziation zwischen seinem Auftreten und einer HHV-8 Infektion besteht, ist davon auszugehen, daß zukünftig Organspender und möglicherweise auch Blutprodukte auf HHV-8 getestet werden, ähnlich wie zum Beispiel heute obligat nach HIV, Hepatitis B und C getestet wird.

Das KS besitzt eine teilweise stark variierende Dignität. Das nicht endemisch auftretende KS betrifft fast immer immundefiziente Patienten und hat in der Regel einen malignen Verlauf. Es wird mit mäßigem Erfolg chemotherapeutisch (z.B. liposomales Doxorubicin), chirurgisch oder mittels Strahlentherapie behandelt. Retrospektive Studien lassen auf eine Wirksamkeit der Virostatika Foscarnet und Ganzyklovir schließen. Größere prospektive Studien zur Wirksamkeit von Virostatika bei KS sind bisher nicht publiziert. Ein einheitliches Behandlungsschema gibt es für die B-Zell Lymphome, in denen HHV-8 gefunden wurde, bisher ebenfalls nicht. Meist wurden Chemotherapieschemata angewandt, die üblicherweise zur Behandlung von Non-Hodgkin-Lymphomen verwendet werden.

Es ist daher eine Aufgabe der vorliegenden Erfindung, neue Mittel zur Diagnostik und Therapie von HHV-8-Infektionen und von HHV-8 direkt oder indirekt verursachter Erkrankungen bereitzustellen.

Diese Aufgabe wird erfindungsgemäß durch die im Anspruch 1 gekennzeichneten monoklonalen Antikörper sowie die in den nebengeordneten Patentansprüchen näher gekennzeichneten Hybridom-Zellinien, Diagnostik-Systeme, Nachweisverfahren und Verwendungen gelöst. Bevorzugte Ausgestaltungen der Erfindung ergeben sich aus den Unteransprüchen.

Erfindungsgemäß werden monoklonale Antikörper bereitgestellt, die spezifisch ein Epitop des Kaposin-Proteins oder ein Derivat hiervon erkennen und an diese Epitope binden. Unter 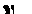 Epitop" wird erfindungsgemäß eine antigene Determinante der Kaposin-Peptide oder eines Derivats hiervon verstanden, die von einem monoklonalen Antikörper erkannt werden und an die der monoklonale Antikörper binden kann.

Das Derivat des Kaposin-Peptids umfaßt eine Aminosäuresequenz des Kaposin-Peptids, die durch Deletion, Substitution, Insertion, Addition und/oder chemische Modifikation einer oder mehrerer Aminosäuren entstanden ist, mit der Maßgabe, daß der gegen ein Epitop des Kaposin-Peptid-Derivats gerichtete Antikörper zum spezifischen Nachweis einer HHV-8-Infektion einsetzbar ist.

Der erfindungsgemäß bereitgestellte monoklonale Antikörper erkennt ein Epitop auf dem zytoplasmatischen Bereich oder dem extrazellulären Bereich des Kaposin-Peptids oder eines Derivats hiervon.

Die in der vorliegenden Erfindung offenbarten monoklonalen Antikörper sind spezifisch gegen ein Epitop eines der nachfolgenden Peptide gerichtet:
(a) AIPPLVCLLA oder
(b) QRGPVAFRTRVATG

In einer weiteren Ausführungsform wird auch die nachfolgende Sequenz QRGPVAFRTRVA mitumfaßt, die sich aus den Peptiden 15 und 16 der Tabelle 1 zusammensetzt.

In einer weiteren Ausführungsform der Erfindung ist der monoklonale Antikörper gegen ein Derivat hiervon mit einer Länge von mindestens 5, mindestens 6 oder mindestens 7 Aminosäuren gerichtet.

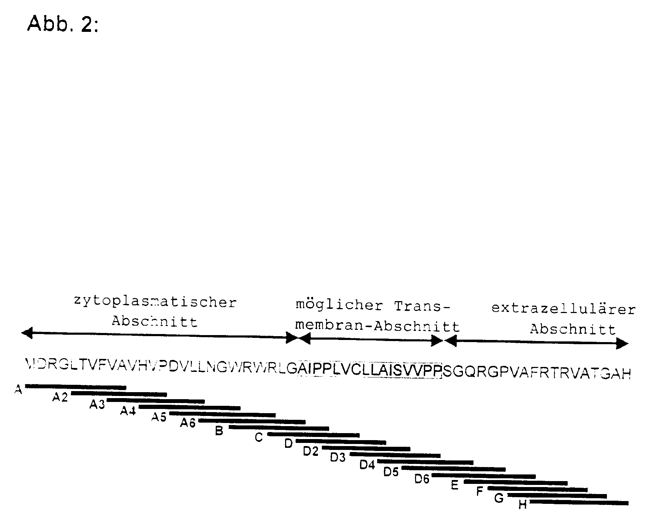

Unter 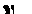 Kaposin-Protein" werden erfindungsgemäß ein Protein mit der in Abb. 2 gezeigten Aminosäuresequenz oder Teilbereiche des Proteins (Peptide) verstanden. Von der Erfindung erfaßt werden die oben aufgeführten Peptide und Derivate davon mit einer Aminosäuresequenz, die durch Deletion, Substitution, Insertion, Addition und/oder chemische Modifikation einer oder mehrerer Aminosäuren entstanden sind, mit der Maßgabe, daß das Derivat zum spezifischen Nachweis einer HHV-8-Infektion einsetzbar ist und/oder mit der Maßgabe, daß gegen das Kaposin-Peptid-Derivat ein monoklonaler Antikörper gerichtet werden kann, der zur Diagnostik und/oder Therapie von HHV-8-Infektionen und - Erkrankungen einsetzbar ist.

Die Erfindung betrifft auch Hybridom-Zellinien, die einen monoklonalen Antikörper produzieren, wie er vorstehend näher gekennzeichnet wurde.

Erfindungsgemäß werden auch Diagnostik-Systeme in Kit-Form bereitgestellt. Diese Diagnostik-Systeme liegen in zwei Ausgestaltungen vor.

In der ersten Ausgestaltungsform dient das Diagnostik-System zum Nachweis des Vorliegens eines Kaposin-Proteins oder eines Derivats hiervon, wie es oben definiert wurde. Im Diagnostik-System liegt mindestens ein monoklonaler Antikörper, wie er oben näher definiert wurde, in einem Behälter vor. Die Kit-Systeme umfassen typischerweise den Antikörper in markierter oder nicht-markierter Form, Reagenzien, um die notwendigen Inkubationen vorzunehmen und Substrate oder Derivatisierungsmittel, die in Abhängigkeit von der verwendeten Markierung eingesetzt werden.

Der Nachweis auf das Vorliegen bzw. die Expression des Kaposin-Proteins oder eines Derivats hiervon in einer biologischen Probe findet beispielsweise wie folgt statt: Die Probe wird mit einem monoklonalen Antikörper, wie er vorstehend beschrieben wurde, unter solchen Bedingungen in Kontakt gebracht, daß sich der Antikörper an einen antigenen Bestandteil der Probe binden kann; anschließend wird der Anteil der Bindung des Antikörpers an den antigenen Bestandteil der Probe bestimmt.

Als Probe wird beispielsweise eine Körperflüssigkeit, eine intakte Zelle, ein Zellextrakt oder ein Gewebe verwendet. Der Anteil der Bindung des Antikörpers kann beispielsweise durch immunzytochemische oder immunhistochemische Färbung bestimmt werden.

In einer zweiten Ausgestaltungsform der Erfindung wird das Diagnostik-System so ausgestaltet, daß es befähigt ist, monoklonale oder polyklonale Antikörper nachzuweisen, die in einer Probe, beispielsweise einer Körperflüssigkeit, einer intakten Zelle, einem Zellextrakt oder einem Gewebe, vorliegen. Hierzu wird ein Kaposin-Peptid oder ein Derivat hiervon, wie es oben und in Anspruch 1 definiert wurde, in einem geeigneten Behältnis in geeigneter Form bereitgestellt, wobei das Protein oder das Derivat hiervon in der Lage ist, eine Immunreaktion mit einem nachzuweisenden monoklonalen oder polyklonalen Antikörper einzugehen. Die Probe wird mit dem Kaposin-Peptid oder einem Derivat hiervon unter geeigneten Bedingungen in Kontakt gebracht, um eine Bindung des in der Probe vorhandenen oder möglicherweise vorhandenen, gegen das Kaposin-Peptid oder einem Derivat hiervon gerichteten Antikörpers zu ermöglichen; weiterhin wird der Anteil der Bindung des Antikörpers in der Probe an das Kaposin-Peptid oder des Derivats hiervon bestimmt.

Die erfindungsgemäßen Verfahren umfassen somit im wesentlichen die Inkubation der zu testenden Probe mit monoklonalen Antikörpern oder, umgekehrt, die Inkubation des Kaposin-Peptids hiervon mit einer zu testenden Probe, die möglicherweise monoklonale Antikörper oder polyklonale Antikörper enthält, die gegen ein Kaposin-Peptid oder ein Derivat hiervon gerichtet sind. Unter 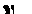 Inkubation" kann erfindungsgemäß auch jede andere Möglichkeit verstanden werden, Probenmaterial mit Antikörpern in Kontakt zu bringen. Zur Anwendung sind viele mögliche grundlegende Verfahren, um derartige Immonoassays durchzuführen, bekannt. Diese Verfahren umfassen beispielsweise RIA, ELISA, Präzipitationsverfahren, Agglutinationsverfahren, Komplement-Fixierungsverfahren und Immunfluoreszenzverfahren. Die monoklonalen Antikörper oder das Kaposin-Peptid oder ein Derivat hiervon können in markierter Form vorliegen. Die Markierungen, die zur Herstellung markierter Formen der Antikörper oder der Proteine eingesetzt werden, umfassen Reste, die direkt nachweisbar sind, beispielsweise Radiomarkierungen und Fluorochrome, als auch Reste, beispielsweise Enzyme, die zum Nachweis umgesetzt oder derivatisiert werden müssen. Die Radiomarkierung kann durch beliebige, bekannte Zellverfahren nachgewiesen werden. Eine Enzymmarkierung kann durch beliebige, an sich bekannte kalorimetrische, spektrophotometrische, fluorospektro-photometrische oder gasometrische Techniken nachgewiesen werden. Das Enzym wird mit dem Antikörper über Brückenmoleküle, beispielsweise Carbodiimide, Periodate, Diisocyanate, Glutaraldehyde, verbunden. In diesen Verfahren können viele, an sich bekannte Enzyme eingesetzt werden. Beispiele hierfür sind Peroxidase, alkalische Phosphatase, β-Glucuronidase, β-D-Glucosidase, β-D-Galactosidase, Urease, Glucose Oxidase plus Peroxidase, Galactose Oxidase plus Peroxidase und Saure Phosphatase. Einsetzbare fluoreszierende Materialien umfassen beispielsweise Fluorescein und seine Derivate, Rhodamin und seine Derivate Auramin, Luciferin, Meerettichperoxidase, akalische Phosphatase Lysozym und Glucose-6-Phosphat-Dehydrogenase. Die Antikörper können mit diesen Markierungen durch an sich bekannte Verfahren verbunden werden. Beispiele für Kopplungsmittel sind Aldehyde, Carbodiimide, Dimaleimide, Imidate, Succinimide um die Antikörper mit den oben beschriebenen fluoreszierenden-, chemolumineszierenden- und Enzymmarkierungen zu versehen.

Die Antikörper und die markierten Antikörper und die hier dargestellten Peptide können verwendet werden, um durch HHV-8 direkt oder indirekt verursachte Erkrankungen, beispielsweise B-Zell-Lymphome und Kaposi-Sarkome, in einem Patienten nachzuweisen und/oder den Status der Erkrankung zu verfolgen. Hierzu sind entweder qualitative oder quantitative Immunoassay-Verfahren einsetzbar. Die Testtechniken umfassen dirkte und indirekte Nachweisverfahren. Falls die Probe beispielsweise Kaposi-Sarkomzellen enthält, wird der markierte Antikörper an diese Zellen binden. Nach dem Waschen des Gewebes oder der Zellen zur Entfernung von nicht-gebundenem markierten Antikörper wird die Probe auf markierte Immunkompexe untersucht. Bei indirekten Nachweisverfahren wird das Gewebe oder die Zellprobe oder eine Körperflüssigkeit mit unmarkiertem monoklonalen Antikörper inkubiert. Die Probe wird dann mit einem markierten Antikörper, der gegen den monoklonalen Antikörper gerichtet ist, behandelt, gewaschen und das Vorliegen ternärer Komplexe wird untersucht.

Zu diagnostischen Zwecken liegen die Antikörper, aber auch die Kaposin-Peptide, typischerweise in Kit-Form vor.

### Zur Beschreibung therapeutischer Anwendungen der Antikörper

Bei einer in vivo-Therapie werden die Antikörper der vorliegenden Erfindung in therapeutischwirksamen Mengen (d.h. Mengen, die durch eine HHV-8-Infektion direkt oder indirekt verursachte Erkrankungen beseitigen oder verringern) an einen Patienten verabreicht. Dies kann normalerweise parenteral oder intravenös erfolgen. Die Dosis und die Formulierung sind von der Natur der zu behandelnden Erkrankung und vom Patienten abhängig.

Das erfindungsgemäß eingesetzte Kaposin-Peptid-Derivat oder das erfindungsgemäß nachzuweisende Kaposin-Protein-Derivat umfaßt bevorzugt eine Aminosäuresequenz des Kaposin-Peptids, die durch Deletion, Substitution, Insertion, Addition und/oder chemische Modifikation einer oder mehrerer Aminosäuren entstanden ist. Das Endprodukt eines derartigen Derivatisierungsprozesses ist jedoch immer so ausgelegt, daß ein spezifischer Nachweis einer HHV-8-Infektion oder einer durch HHV-8-Infektion induzierten Erkrankung möglich ist.

In der Erfindung umfaßt das Kaposin-Peptid ein Epitop auf dem zytoplasmatischen Bereich und dem extrazellulären Bereich des Kaposin-Proteins oder eines Derivats hiervon.

Die Fragmente des Kaposin-Peptids weisen eine Länge von mindestens 5, mindestens 6 oder mindestens 7 Aminosäuren auf. Die erfindungsgemäß bereitgestellten monoklonalen Antikörper richten sich gegen ein Kaposin-Peptid oder die oben näher beschriebenen Derivate des Kaposin-Peptids.

Die Antikörper der vorliegenden Erfindung werden zur Diagnostik und zur Therapie von Erkrankungen eingesetzt, die durch Infektionen mit HHV-8 direkt oder indirekt verursacht sind. Dazu gehören beispielsweise das Kaposi-Sarkom, AIDS-Erkrankungen und B-Zell-Lymphome.

Auch die erfindungsgemäß bereitgestellten Kaposin-Peptide oder die oben näher bezeichneten Derivate der Kaposin-Peptide werden zur Diagnostik und Therapie von HHV-8-Infektionen, AIDS-Erkrankungen, Kaposi-Sarkomen und B-Zell-Lymphomen eingesetzt.

Die Aminosäuresequenz der Peptide ist wie folgt:
(a) AIPPLVCLLA
(b) QRGPVAFRTRVATG

In einer weiteren Ausführungsform ist die Sequenz wie folgt:
QRGPVAFRTRVA

Nachfolgend wird die Erfindung anhand von Ausführungsbeispielen und anhand der beiliegenden Abbildungen näher beschrieben.

Die Abbildungen zeigen:
**Abb. 1: Reaktivität von Seren von Patienten mit Kaposi Sarkom gegen das rekombinante *minor capsid protein* VP23**
   (a) Da*s minor capsid protein* VP23 von HHV-8 wurde als GST-getaggtes Protein in E.coli exprimiert, per Affinitätschromatographie mittels Glutathionsepharose aufgereinigt und im ELISA zum Nachweis von anti-VP23 Antikörpern in Patientenseren eingesetzt. (b) Die Mikrotiterplaten wurden mit 1 µg rekombinantem VP23 beschichtet und mittels PBS/0,1% Tween 20 geblockt. Die Seren von 19 HIV-negativen Kontrollspendern, 19 HIV-positiven Individuen ohne KS und 36 HIV-negativen Patienten mit KS wurden mit lyophilisierten *E.coli* präinkubiert und anschließend in einer Verdünnung von 1:50 2 Stunden bei Raumtemperatur auf der Mikrotiterplatte inkubiert. Nach 4 Waschschritten wurde ein sekundärer, Peroxidasegekoppelter Antikörper und danach ABTS Substrat zugegeben. Die Seren der HIV-positiven KS Patienten zeigen deutlich Reaktivität gegen VP23 (Wilcoxon Test p<0.0024), doch nur etwa 1/3 der Seren liegt über einem Cutoff von OD 0,2. Der Test eignet sich deshalb kaum zum Nachweis einer HHV-8 Infektion.
**Abb. 2: Aminosäuresequenz des ersten orf der T0.7 RNA (** 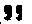 **Kaposin")**
   In der Aminosäuresequenz von *Kaposin* ist die potentielle Transmembranregion (dunkel unterlegt), die Bindungsstelle des mAB kap5C4 (schraffiert unterlegt) und eine zweite hydrophobe Region (hell unterlegt) mit bisher unbekannter Funktion gekennzeichnet. Die zur Epitopkartierung verwendeten Peptide (siehe auch Tabelle 1) sind im unteren Teil der Abbildung gezeigt.

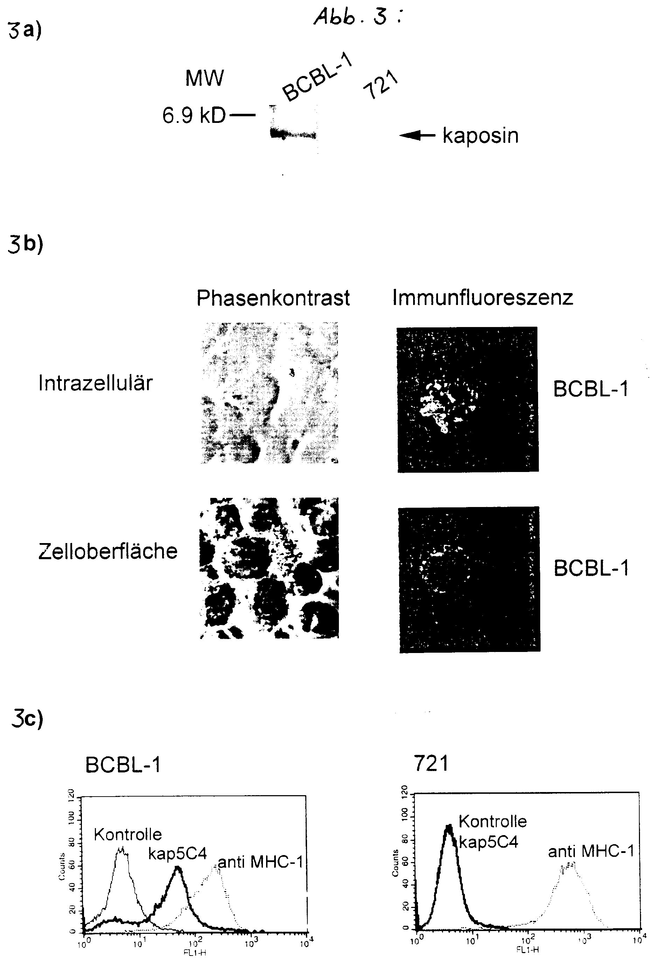

**Abb. 3: Nachweis der Expression von *Kaposin* in der HHV-8+ Zellinie BCBL-1 mit Hilfe des mAB kap5C4**
   (a) Nachweis von *Kaposin* per Western Blot in nicht induzierten HHV-8+ BCBL-1 Zellen, jedoch nicht in der EBV+ Kontrollzellinie 721. (b) Nachweis von *Kaposin* in fixierten/permeabilisierten (oben) und nicht permeabilisierten (unten) BCBL-1 Zellen mittels Immunfluoreszenz und Auswertung mittels Konfokalem Lasermikroskop. In den beiden linken Abbildungen sind die rechts in der Immunfluoreszenz gezeigten Zellen mittels Phasenkontrastaufnahme wiedergegeben. Die nicht induzierten BCBL-1 Zellen wurden zunächst mit unverdünntem Zellkulturüberstand des kap5C4 Hybridoms und anschließend mit einem Cy-3 markierten, sekundärem Antikörper reagiert. (c) Auswertung der oben gezeigten, mit kap5C4 angefärbten Zellen im Flowzytometer (breite Kurve). Als Positivkontrolle wurde ein gegen MHC Klasse 1 gerichteter Antikörper W6/32 verwendet (gestrichelte Kurve). Bei der Negativkontrolle wurde nur mit dem sekundären Antikörper inkubiert (einfache Kurve).

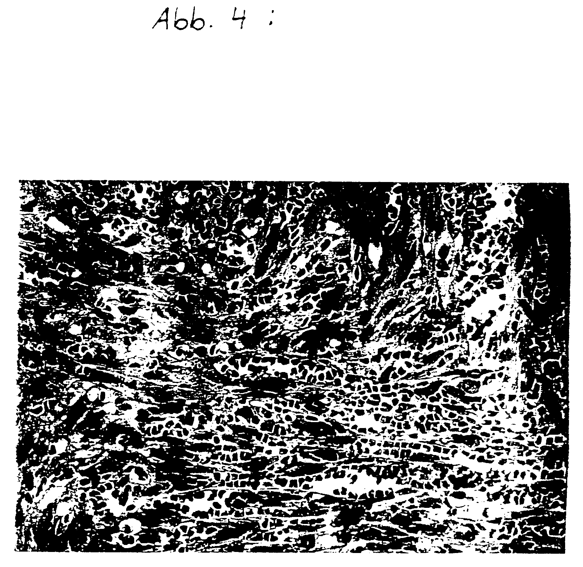

**Abb. 4: Immunhistologische Färbund von KS Gewebe mit dem mAB gegen das lytische HHV-8 Protein VP23**
   Der mit Formalin fixierte und in Paraffin eingebettete Dünnschnitt eines Kaposi Sarkoms wurde mit dem mAB vp4G2 gegen HHV-8 VP23 angefärbt. Der Schnitt wurden zunächst durch Kochen von Paraffin befreit und anschließend mit einer 1:10 Verdünnung des mAB vp4G2 inkubiert. Gebundene Antikörper wurden mit Hilfe des EnVision™ Systems (Dako, Hamburg) nachgewiesen.

Latent HHV-8 infizierte Zellen exprimieren wenigstens 3 virale Transkripte (siehe 4.). Die in der Latenzphase häufigste virale mRNA T0.7 hat eine Länge von 0,7 kb und besitzt zwei kurze offene Leserahmen (orf). Bisher wurde nicht gezeigt, daß einer dieser orfs translatiert wird. Der erste orf besitzt nur eine Länge, die 60 Aminosäuren entspricht. In der gesamten Literatur sind insgesamt nur 2 Transmembranproteine beschrieben, die kleiner als 14 kd sind (*ponticulin* und *L-selectin*), weshalb nicht klar war, ob T0.7 überhaupt translatiert wird und nicht wie die das gleichzeitig entdeckte T1.1/nut-1 Transkript (welches ebenfalls einen orf mit einer Länge von 62 Aminosäuren kodiert) eine regulatorische Funktion besitzt. Die Patentanmeldung hat folgende Untersuchungen des ersten orfs von T0.7, genannt 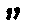 *Kaposin"*, zur Grundlage:
*1. Expression des ersten orfs des T0.7 Transkripts (* 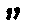 *Kaposin") in HHV-8 infizierten Zellen*
   Der Antragsteller konnte zeigen, daß der erste offene Leserahmen des T0.7 Transkripts, 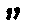 *Kaposin"*, tatsächlich in HHV-8 infizierten Zellen translatiert wird. Die Sequenz des ersten orfs wurde in *E.coli* als GST-Fusionsprotein (Glutathion-S-Transferase) exprimiert und affinitätschromatographisch aufgereinigt. Das rekombinante Fusionsprotein wurde zur Herstellung von monoklonalen Antikörpern (mAB) verwendet. Hierzu wurden Lou/C Ratten immunisiert, und die Milzzellen nach Standardmethoden mit der Myelomzellinie P3C63Ag8.653 fusioniert. Die Antikörper wurden per ELISA mit rekombinantem Fusionsprotein gescreent und mit rekombinantem GST gegengescreent. Der mAB kap5C4 erkennt im Western Blot rekombinantes *Kaposin*-Fusionsprotein, nicht jedoch GST. Mittels Peptidmapping wurde herausgefunden, daß der mAB kap5C4 die Peptidsequenz QRGPVAFRTRVA am C-Terminus erkennt (Abb. 2 und Tab. 1). In Zell-Lysaten von HHV-8 infizierten Zellinien konnte mittels Western Blot, Immunpräzipitation und nach Oberflächenbiotinylierung ein Protein identifiziert werden, welches die aufgrund der Sequenz vorhergesagte Länge von 6 kd besitzt (Abb. 3a)
*2. Expression von Kaposin an der Zelloberfläche*
   Mittels Immunfluoreszenz wurde gezeigt, daß *Kaposin* auf der Zelloberfläche von HHV-8 infizierten Zellinien exprimiert wird (Abb. 3b und c). Die Abbildung 3b zeigt, daß *Kaposin* in fixierten und permeabilisierten BCBL-1 Zellen (HHV-8 positive, EBV negative Zellinie, die aus einem Primären Effusionslymphom etabliert wurde) von dem mAB kap5C4 erkannt wird. Die Abbildung zeigt außerdem, daß *Kaposin* auch auf nicht permeabilisierten Zellen nachweisbar ist und sich deshalb auf der Zelloberfläche befinden muß. Die Immunfluoreszenz zeigt eine randsaumartige Färbung wie sie für Oberflächenmoleküle typisch ist. Die Abbildung 3c zeigt eine flowzytometrische Analyse von BCBL-1 Zellen, die mit kap5C4 bzw. einem Kontrollantikörper gegen MHC Klasse 1 angefärbt wurden. Man erkennt, daß die Expression mit dem mAB kap5C4 deutlich positiv etwa eine Logstufe über der Negativkontrolle ist. Da kap5C4 das C-terminale Ende von *Kaposin* erkennt, sind die Ergebnisse so zu interpretieren, daß Kapsin ein Typ II Protein ist (N-Terminus befindet sich im Zytoplasma).
*3. Expression von Kaposin in der Mehrzahl latent HHV-8 infizierter Zellen*
   Lytische Proteine, wie zum Beispiel das vom Antragsteller untersuchte VP23 *minor capsid protein*, werden von HHV-8+ Zellinien nur nach Induktion mit Phorbolester und/oder n-Butyrat exprimiert. In der Immunhistologie ist in den meisten KS Geweben nur ein sehr geringer Bruchteil der Spindelzellen positiv mit dem mAB vp4G2 gegen VP23 (Abb. 4). Im Gegensatz hierzu exprimieren HHV-8+ Zellinien *Kaposin* konstitutiv ohne vorherige Induktion. Je nach Zellinie und Färbemethode variiert der Prozentsatz positiver Zellen zwischen 10 und über 80%. Auch in der Immunhistologie ist die Anzahl mit dem mAB kap5C4 angefärbter Zellen weitaus größer als die vp4G2 positiven Zellen. Wahrscheinlich ist mit einer sensitiven Färbemethode der überwiegende Teil aller Spindelzellen positiv für *Kaposin*.
*4. Erkennung von Kaposin durch Seren von KS Patienten und KS Risikogruppe*n
   Mittels Western Blot konnte gezeigt werden, daß *Kaposin*-Fusionsprotein von Seren von KS Patienten bzw. von HIV-Infizierten ohne KS erkannt wird. Es zeigte sich, daß ein höherer Prozentsatz (ca. 80%) der Seren von HIV-Infizierten (Risikogruppe: Homosexuelle) mit *Kaposin* reagierten als gegen andere virale Proteine/Peptide. Dies könnte ein Hinweis darauf sein, daß sich *Kaposin* als latentes virales Membranprotein besser zum Nachweis früher Infektionen eignet als lytische Proteine. Mittels Peptid-ELISA wurde eine Grobkartierung zur Bestimmung des von diesen Seren erkannten Epitops durchgeführt. Es zeigte sich, daß ein hydrophobes Peptid mit der Aminosäuresequenz AIPPLVCLLA von den meisten dieser Seren erkannt wurde (Tab. 1).

### Vorteile gegenüber dem bisherigen Stand der Technik und potentielle Nutzung

Wie oben dargelegt, sind die derzeitigen Nachweismethoden einer HHV-8 Infektion mit zahlreichen Nachteilen behaftet. Eine spezifisch gegen HHV-8 gerichtete Therapie existiert im Moment nicht. Das hier beschriebene virale Protein *Kaposin* besitzt gegenüber den bisher verwendeten viralen Proteinen folgende Vorteile:
1. Expression an der Zelloberfläche
2. Expression in der Mehrzahl der HHV-8 infizierten Zellen
3. *Kaposin* ist das am besten exprimierte latente Protein von HHV-8
4. Homologien zu anderen Proteinen (auch viralen) sind bisher nicht bekannt

Die potentiellen Nutzen ergeben sich folglich aus der Funktion von *Kaposin* als zuverlässiger *Infektions*- oder *Tumormarker*, welcher nach den vorliegenden Daten von einer Mehrzahl der HHV-8 infizierten Zellen gut exprimiert wird. *Kaposin* eignet sich daher zur Entwicklung (i) neuer diagnostischer Verfahren zum Nachweis einer HHV-8 Infektion und (ii) von spezifisch gegen HHV-8 gerichteten Therapeutika. Im diagnostischen Bereich ist z.B. ein Einsatz von rekombinantem *Kaposin* in ELISAs oder Western Blots zum serologischen Nachweis oder die Verwendung von mAB gegen *Kaposin* zum Nachweis von HHV-8 infizierten Zellen in der Immunhistologie denkbar. Ein zuverlässiger serologischer Test hat die folgenden Anwendungsmöglichkeiten:
- Diagnose einer HHV-8 Infektion;
- prognostischer Marker für die Entwicklung eines KS-Sarkoms bei HIV-Infizierten, Transplantierten und immunsupprimierten Patienten;
- Verlaufsparameter bei der Therapie des Kaposi Sarkoms;
- Diagnose bestimmter B-Zell Lymphome;
- Verlaufsparameter bei der Therapie der HHV-8+ B-Zell Lymphome;
- evtl. Diagnose bisher nicht bekannter Tumore (z.B. auch sogenannter 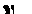 Zweittumore" nach Organtransplantation).

Auch eine therapeutische Nutzung von *Kaposin* ist prinzipiell denkbar, z.B. beim Einsatz von bispezifischen Antikörpern, die durch Vernetzung von HHV-8+ Tumorzellen mit T-Lymphozyten zu einer zielgerichteten Aktivierung der Immunantwort und damit zu einer Elimination der Tumorzellen führen könnten. Da bisher nicht bekannt ist, durch welchen Mechanismus HHV-8 infizierte Zellen transformiert werden, könnte *Kaposin* als latentes Protein dabei ursächlich beteiligt sein. Sollte sich dies bewahrheiten, so könnte eine therapeutische Intervention, die zu einer Blockade von *Kaposin* führt, einen therapeutischen Nutzen haben.

### Zellinien

Die verwendeten Zellinien BCBL-1 (HHV-8+, EBV-) (freundlicherweise zur Verfügung gestellt von Don ganem; UCSF, USA) und 721 (EBV+ lymphoblastoide Zellinie) wurden in RPMI (Life Technologies, Paisley, UK) supplementiert mit 20 % Hitze-inaktiviertem fötalen Kälberserum, 100 lU(ml Penicillin, 100 µg/ml Strptomycin, 2 mM L-Glutamin und 0,05 mM 2-Mercaptoethanol (Sigma, St. Louis, USA) passagiert.

### Herstellung von rekombinanten HHV-8 Fusionsproteinen

Die Sequenz des ersten orfs von T0.7 wurde entsprechend Standardnethoden aus extrahiertrer DNA von BCBL-1 Zellen mittels PCR amplifiziert, in den Vektor pGEX-4T (Pharmacia, Uppsala, Schweden) kloniert, in E.coli als GST-Fusionsprotein (Glutathion-S-Transferase) exprimiert und affinitätschromatographisch aufgereinigt (Ausubel et al. 1992).

### Herstellung monoklonaler Antikörper gegen Kaposin

Die monoklonalen Antikörper wurden nach Standardmethoden, wie bereits beschrieben, hergestellt (Kremmer et al., 1995). Lou/C Ratten wurden in Abständen von 3 Wochen mit dem rekombinanten Fusionsprotein und Trehalose Dimycolate/Monophosphoryl Lipid A/Squalen/Tween 80 (Antibody Multiplier ABM-S, Linaris, Bettingen, Germany) als Adjuvans insgesamt dreimal immunisiert. Die Milzzellen der immunisierten ratten wurden mit der Myelomzellinie P3C63Ag8.653 fusioniert und die Hybridomüberstande mittels ELISA (Enzyme-Linked Immunosorbent Assay) auf Mikrotiterplatten, die mit dem rekombinanten Protein in einer Konzentrastion von 1 µg/well beschichtet waren, getestet. Die Überstände wurden 1 Stunde inkubiert und die gebundenen Antikörper mit Hilfe eines sekundären, Peroxidase-konjugierten Antikörpers gegen Ratten Immunglobulin nachgewiesen. Danach wurde o-Phenylenediamin als Substrat zugegeben und die Extinktion in einem ELISA-Reader (Tecan, Reading, UK) gemessen. Der Antikörper kap5C4 wurde insgesamt zweimal kloniert.

### Western Blotting

Zellysate von unstimulierten BCBL-1 und 721 Zellen wurden auf einem 18 % SDS_PAGE aufgetrennt und auf Nitrozellulose geblottet (Schleicher and Schuell, Dassel, Germany). Anschließend wurde der Blott mit 5 % Milchpulver (Merck, Darmstadt, Germany) in TBS /Tris balanced saline)/0,02 % Tween-20 geblockt und mit dem mAB kab5C4 inkubiert. Nach drei Waschschritten in TBS/0,02 % Tween-20 wurde der Blot eine Stunde mit einem Alkalische Phosphatase-konjugiertem sekundären Antikörper (Dianova, Hamburg, Germany) in einer Verdünnung von 1 : 1000 inkubiert, nochmals gewaschen und schließlich mit Western Blue (Promega, Madison, USA) nach den Angaben des Herstellers inkubiert.

### Immunfluoreszenz

Zur intrazellulären Färbung wurden die nicht-induzierten BCBL-1 Zellen mit Aceton fixiert und mit 0,2 % Saponin permeabilisiert. Zur Oberflächenfärbung wurden unbehandelte BCBL-1 Zellen verwendet. Die BCBL-1 Zellen wurden zunächst mit unverdünntem Zellkulturüberstand des kap5C4 Hybridoms und anschließend mit einem Cy-3 markierten, sekundärem Antikörper (Sigma, St. Louis, USA) inkubiert. Zur Analyse wurden die Zellen auf Poly-L-Lysine beschichteten Objekttragern (Marienfeld, bad Mergentheim, Germany) fixiert und mittels eines Leica TNT Konfokalen Lasermikroskops (Leica, Bensheim, Germany) analysiert. Zur Kontrolle in der Flowzytometrie wurde der mAB W6/32 gegen MHC Klasse 1 als Primärantikörper verwendet. Die Analyse wurde auf einem FACSscalibur Flowzytometer (Becton, Dickinson, Mountain View, USA) durchgeführt.

### ELISA

Die Mikrotiterplatten wurden mit 1 µg rekombinanten VP23 beschichtet und mittels PBS/0,1% Tween-20 geblockt. Die Seren von 19 HIV-negativen Kontrollspendern, 19 HIV-positiven Individuen ohne KS und 36 HIV-negativen Patieneten mit KS wurden mit lyophilisierten E.coli präinkubiert und anschließend in einer Verdünnung von 1 : 50 zwei Stunden bei Raumtemperatur auf der Mikrotiterplatte inkubiert. Nach 4 Waschschritten wurde ein sekundärer, Peroxidase-gekoppelter Antikörper und danach ABTS Substrat (Boehringer, Mannheim, Germany) zugegeben.

Zur Durchführung des Peptid-ELISA's wurden Peptide verwendet, die N-terminal mit einem SGSG-Spacer versehen und biotinyliert sind. Eine Stammlosung der jeweiligen Peptide (Konzentration 1 µmol/ml in 100 % DMSO) wurde 1 400 in PBS/5 % Tween-20 verdünnt und an Streptavidin-beschichtete Mikrotiterplatten gebunden. Nach einer Inkubation mit einer 1 . 200 Verdünnung des mAB kap5C4 Hybridomüberstands bzw. 1 . 50 des KS Patientenserums wurde ein Peroxidase-konjugierter sekundärer Antikörper gegen IgG der Ratte (kap5C4) bzw. des Menschen in einer verdünnung von 1 . 10000 verwendet. Nach der Reaktion mit dem sekundären Antikörper wurden die Mikrotiterplatten mit ABTS Substrat inkubiert und anschließend in einem ELISA Reader bei einer Wellenlänge von 492 nm gemessen. Zur Identifikation der Epitope, gegen die die Antikörper in HHV-8 infizierten Personen gerichtet sind, wurde ein Serumpool aus HIV-positiven Patienten (<10) mit Kaposi Sarkom verwendet.

### Immunhistologie

Der Schnitt wurde zunächst durch Kochen von Paraffin befreit und anschließend mit einer 1 : 10 Verdünnung des mAB vp4G2 inkubiert. Gebundene Antikörper wurden mit Hilfe des EnVision™ Systems (Dako, Hamburg, Germany) nachgewiesen

**Tabelle 1**

| **Epitopkartierung des monoklonalen Antikörpers kap5C4 und von KS Patientenseren** | | | | |
|---|---|---|---|---|
| | **Bezeichnung** | **Peptidsequenz (N→C)** | **Reaktivität mAB kap5C4** | **Reaktivität KS Patientenseren** |
| 1. | K12A | MDRGLTVFVA | - | - |
| 2. | K12A2 | LTVFVAVHVP | - | - |
| 3. | K12A3 | VAVHVPDVLL | - | - |
| 4. | K12A4 | HVPDVLLNGW | - | - |
| 5. | K12A5 | DVLLNGWRWR | - | - |
| 6. | K12A6 | LNGWRWRLGA | - | - |
| 7. | K12B | WRWRLGAIPP | - | - |
| 8. | K12C | RLGAIPPLVC | - | - |
| 9. | K12D | AIPPLVCLLA | - | +++ |
| 10. | K12D2 | PLVCLLAISV | - | - |
| 11 | K12D3 | CLLAISVVPP | - | - |
| 12. | K12D4 | AISVVPPSGQ | - | - |
| 13. | K12D5 | VVPPSGQRGP | - | - |
| 14. | K12D6 | PSGQRGPVAF | - | - |
| 15. | K12E | QRGPVAFRTR | +++ | - |
| 16. | K12F | GPVAFRTRVA | +++ | - |
| 17. | K12G | VAFRTRVATG | - | - |
| 18. | K12H | FRTRVATGAH | - | - |
| Die angegegebenen Peptide sind N-terminal mit einem SGSG-Spacer versehen und biotinyliert. Eine Stammlösung der jeweiligen Peptide (Konzentration 1 µmol/ml in 100% DMSO) wurde 1:400 in PBS/5% Tween 20 verdünnt und an Streptavidin-beschichtete Mikrotiterplatten gebunden. Nach einer Inkubation mit einer 1:200 Verdünnung des mAB kap5C4 Hybridomüberstands bzw. 1:50 des KS Patientenserums wurde ein Peroxidase-konjugierter sekundärer Antikörper gegen IgG der Ratte (kap5C4) bzw. des Menschen in einer Verdünnung von 1:10.000 verwendet. Nach der Reaktion mit dem sekundären Antikörper wurden die Mikrotiterplatten mit ABTS Substrat inkubiert und anschließend in einem ELISA Reader bei einer Wellenlänge von 492 nm gemessen. Zur Identifikation der Epitope, gegen die die Antikörper in HHV-8 infizierten Personen gerichtet sind, wurde ein Serumpool aus HIV-positiven Patienten (>10) mit Kaposi Sarkom verwendet. | | | | |

### Literatur:

Boshoff, C. and Weiss, R.A. (1997). Aetiology of Kaposi's sarcoma: current understanding and implications for therapy. Mol. Med. Today 488-494.
Chang, Y., Cesarman, E., Pessin, M.S., Lee, F., Culpepper, J., Knowles, D.M., and Moore, P.S. (1994). Identification of herpesvirus-like DNA sequences in AIDS-associated Kaposi's sarcoma. Science *266*, 1865-1869.
Neipel, F., Albrecht, J.-C., and Fleckenstein, B. (1997). Cell-homologous genes in the Kaposi's sarcoma-associated rhadinovirus human herpesvirus 8: determinants of its pathogenicity? J. Virol. *71*, 4187-4192.
Russo, J.J., Bohenzky, R.A., Chien, M.-C., Chen, J., Yan, M., Maddalena, D., Parry, J.P., Peruzzi, D., Edelman, I.S., Chang, Y., and Moore, P. (1996). Nucleotide sequence of the Kaposi's sarcoma-associated herpesvirus (HHV8). Proc. Natl. Acad. Sci. USA *93*, 14862-14867.
Zhong, W., Wang, H., Herndier, B., and Ganem, D. (1996). Restricted expression of Kaposi sarcoma-associated herpesvirus (human herpesvirus 8) genes in Kaposi sarcoma. Proc. Natl. Acad. Sci. USA *93*, 6641-6646.
Zhong, W. and Ganem, D. (1997). Characterization of ribonucleoprotein complexes containing an abundant polyadenylated nuclear RNA encoded by Kaposi's sarcoma-associated herpesvirus (Human Herpesvirus 8). J. Virol. *71*, 1207-1212.

## Patentansprüche

1. Monoklonaler Antikörper,
dadurch gekennzeichnet, daß
er spezifisch ein Epitop eines der nachfolgenden Kaposin-Peptide oder ein Derivat hiervon erkennt und hieran bindet:
(a) AIPPLVCLLA
(b) QRGPVAFRTRVATG
(c) QRGPVAFRTRVA

2. Monoklonaler Antikörper nach Anspruch 1,
dadurch gekennzeichnet, daß
das Derivat eine Aminosäuresequenz eines der genannten Peptide umfaßt, die durch Deletion, Substitution, Insertion, Addition und/oder chemische Modifikation einer oder mehrerer Aminosäuren entstanden ist, mit der Maßgabe, daß der gegen ein Epitop des Kaposin-Peptid-Derivats gerichtete Antikörper zum spezifischen Nachweis einer HHV-8-Infektion einsetzbar ist.

3. Monoklonaler Antikörper nach Anspruch 1 oder 2,
dadurch gekennzeichnet, daß
er eines der genannten Kaposin-Peptidfragment-Derivate hiervon mit einer Länge von mindestens fünf Aminosäuren erkennt und hieran bindet.

4. Hybridom-Zellinie,
dadurch gekennzeichnet, daß
sie einen monoklonalen Antikörper nach einem oder mehreren der vorhergehenden Ansprüche produziert.

5. Diagnostik-System in Kit-Form,
dadurch gekennzeichnet, daß
es zumindest einen monoklonalen Antikörper nach einem oder mehreren der Ansprüche 1 bis 3 in einem Behälter zum Nachweis auf das Vorliegen eines Kaposin-Proteins oder eines Derivats hiervon umfaßt.

6. Diagnostik-System in Kit-Form,
dadurch gekennzeichnet, daß
es eines der in Anspruch 1, 2 und 3 genannten Kaposin-Peptide oder ein Derivat hiervon umfaßt, wobei das Peptid befähigt ist, eine Immunreaktion mit einem monoklonalen oder polyklonalen Antikörper einzugehen, der gegen das Kaposin-Protein oder ein Derivat hiervon gerichtet ist.

7. Diagnostik-System nach Anspruch 5 oder 6,
dadurch gekennzeichnet, daß
das Derivat des Kaposin-Peptids oder -Proteins eine Aminosäuresequenz umfaßt, die durch Deletion, Substitution, Insertion, Addition und/oder chemische Modifikation einer oder mehrerer Aminosäuren entstanden ist, mit der Maßgabe, daß das Derivat zum spezifischen Nachweis einer HHV-8-Infektion einsetzbar ist.

8. Diagnostik-System nach einem oder mehreren der vorhergehenden Ansprüche 5-7,
dadurch gekennzeichnet, daß
das Kaposin-Peptid- oder -Protein Derivat ein Peptidfragment eines der genannten Kaposin-Peptide mit einer Länge von mindestens fünf Aminosäuren ist.

9. Verfahren zum Nachweis des Vorliegens des Kaposin-Proteins oder eines Derivats hiervon in einer biologischen Probe,
dadurch gekennzeichnet, daß
die Probe mit einem monoklonalen Antikörper nach einem oder mehreren der Ansprüche 1 bis 3 unter solchen Bedingungen in Kontakt gebracht wird, daß der Antikörper an einen antigenen Bestandteil der Probe binden kann und die Bindung des Antikörpers an den antigenen Bestandteil der Probe bestimmt wird.

10. Verfahren nach Anspruch 9,
dadurch gekennzeichnet, daß
als Probe eine Flüssigkeit, eine intakte Zelle, ein Zell-extrakt oder ein Gewebe verwendet wird.

11. Verfahren nach Anspruch 9 oder 10,
dadurch gekennzeichnet, daß
der Anteil der Bindung des Antikörpers durch immunzytochemische oder immunhistochemische Färbung bestimmt wird.

12. Verfahren zum Nachweis von Antikörpern, die gegen das Kaposin-Protein oder ein Derivat hiervon gerichtet sind, in einer biologischen Probe,
dadurch gekennzeichnet, daß
das Verfahren das Inkontaktbringen der Probe mit einem Kaposin-Peptid umfaßt, das eines der nachfolgenden Peptide a), b) und c) oder die Derivate hiervon unter solchen Bedingungen umfaßt, die geeignet sind, eine Bindung des in der Probe vorhandenen, gegen das Kaposin-Protein oder einem Derivat hiervon gerichteten Antikörpers zu ermöglichen, und Bestimmung des Anteils der Bindung des Antikörpers in der Probe an das Kaposin-Peptid oder das Derivat hiervon, wobei a), b) und c) wie folgt definiert sind:
(a) AIPPLVCLLA
(b) QRGPVAFRTRVATG
(c) QRGPVAFRTRVA

13. Verfahren nach Anspruch 12,
dadurch gekennzeichnet, daß
als Probe eine Flüssigkeit, eine intakte Zelle, ein Zell-extrakt oder ein Gewebe verwendet wird.

14. Verfahren nach Anspruch 12 oder 13,
dadurch gekennzeichnet, daß
das Derivat des Kaposin-Peptids eine Aminosäuresequenz umfaßt, die durch Deletion, Substitution, Insertion, Addition und/oder chemische Modifikation einer oder mehrerer Aminosäuren entstanden ist, mit der Maßgabe, daß das Kaposin-Protein-Derivat zum spezifischen Nachweis einer HHV-8-Infektion einsetzbar ist.

15. Verfahren nach einem oder mehreren der vorhergehenden Ansprüche 12 bis 14,
dadurch gekennzeichnet, daß
das Kaposin-Peptid-Derivat ein Peptidfragment mit einer Länge von mindestens fünf Aminosäuren ist.

16. Verwendung eines monoklonalen Antikörpers nach einem oder mehreren der vorhergehenden Ansprüche 1 bis 3 zur Diagnostik und Therapie von Erkrankungen, die direkt oder indirekt durch Infektionen mit HHV-8 verursacht werden.

17. Verwendung nach Anspruch 16,
dadurch gekennzeichnet, daß
der monoklonale Antikörper zur Diagnostik und Therapie des Kaposi-Sarkoms, von Aids-Erkrankungen und B-Zell-Lymphomen eingesetzt wird.

18. Verwendung eines Kaposin-Peptids oder eines Derivats hiervon mit der nachfolgenden Sequenz:
(a) AIPPLVCLLA
(b) QRGPVAFRTRVATG
(c) QRGPVAFRTRVA
zur Diagnostik und Therapie von Erkrankungen, die direkt oder indirekt durch Infektionen mit HHV-8 verursacht werden.

19. Verwendung nach Anspruch 18,
dadurch gekennzeichnet, daß
das Kaposin-Peptid-Derivat eine Aminosäuresequenz a) oder b) umfaßt, die durch Deletion, Substitution, Insertion, Addition und/oder chemische Modifikation einer oder mehrerer Aminosäuren entstanden ist, mit der Maßgabe, daß das Kaposin-Peptid-Derivat zum spezifischen Nachweis einer HVV-8-Infektion einsetzbar ist.

20. Verwendung nach Anspruch 18 oder 19,
dadurch gekennzeichnet, daß
das Kaposin-Peptid-Derivat ein Peptidfragment mit einer Länge von mindestens fünf Aminosäuren ist.

21. Kaposin-Peptid mit einer der nachfolgenden Sequenzen:
(a) AIPPLVCLLA
(b) QRGPVAFRTRVATG
(c) QRGPVAFRTRVA
